# EUROPEAN PATENT APPLICATION

(11) **EP 3 714 884 A1**
(43) Date of publication of application: **30.09.2020**
(21) Application number: 19165517.4
(22) Date of filing: 27.03.2019
(51) Int. Cl.: A61K 31/4468, A61K 9/00, A61K 31/5517, A61K 45/06, A61P 23/00

(54) **MEDICAL USES FOR BENZODIAZEPINES WITH A PARTICULAR EEG PATTERN**

(71) Applicant: Paion UK Limited, Cambridge, Cambridgeshire CB4 2QH (GB)
(72) Inventor: The designation of the inventor has not yet been filed
(74) Representative: Lücke, Robert

(57) **Abstract**

The present invention relates to a benzodiazepine or pharmaceutically acceptable salt thereof for use in inducing and/or maintaining a MOAA/S score of ≤ 1 in a human patient selected from an infant, a child and a patient with a cognitive impairment that is at least mild cognitive impairment, or for use in inducing and/or maintaining a MOAA/S score of ≤ 1 in a patient without causing brain damage, wherein the benzodiazepine or salt is defined by having the property of leading to one or more particular characteristics of an electroencephalogram of a human reference subject in case said benzodiazepine or salt is administered to said reference subject to induce and/or maintain a MOAA/S score of ≤ 1.

## Description

The present invention relates to the field of anaesthesia. In more detail, it relates to a benzodiazepine or salt thereof for use in inducing and/or maintaining a MOAA/S score of ≤ 1 in specific patient groups having a higher risk for damage from cerebral hypotension (more specifically a human patient selected from an infant, a child and a patient with a cognitive impairment that is at least mild cognitive impairment), or for use in inducing and/or maintaining a MOAA/S score of ≤ 1 in a patient without causing brain damage, wherein the benzodiazepine or salt is defined by a certain EEG signature pattern. This EEG signature pattern means that the benzodiazepine or salt has the property of leading to one or more particular characteristics of an electroencephalogram of a human reference subject in case it is administered to said reference subject to induce and/or maintain a MOAA/S score of ≤ 1.

By producing a state of general anaesthesia in a patient, physical and mental pain can be avoided during medical procedures that would otherwise be intolerable. One notable example is surgery. General anaesthesia is defined as unconsciousness (sedation) and the elimination of memory (amnesia) and additionally requires the elimination of pain (analgesia), the loss of body motion (muscle relaxation) and the loss of unwanted protective reflexes (reflex inhibition). It is typically achieved by a combination of anaesthetic drugs

One of the common applications of electroencephalography is in monitoring the depth of anaesthesia (including general anaesthesia). In this non-invasive electrophysiological method, the electrical activity of the patient's brain is measured, with electrodes placed on his/her scalp. What is recorded is called an electroencephalogram (EEG). The oscillations observed are, based on their frequency, subdivided into bands. Various definitions of the exact boundaries of these bands exist. In the present invention, the bands of an electroencephalogram are defined as follows:

| | |
|---|---|
| - delta: | 0.5 to < 2 Hz |
| - theta: | 2 to < 8 Hz |
| - alpha: | 8 to < 13 Hz |
| - beta: | 13 to < 30 Hz |
| - gamma: | 30 to 50 Hz |

In the description of frequency ranges, the less than sign ("<") is used herein to indicate all values immediately up to, but not including the value that follows the sign. Where a particular EEG device uses different boundaries of the EEG bands, this is indicated in the individual case, but does not affect the generality of the above definitions.

Drug effects may be measured by recording an EEG. For example, the frequency bands of an EEG, in particular the theta band, allow conclusions about the effects of a drug on the brainstem. The brainstem controls cardiovascular and respiratory functions. The activity in the alpha band often correlates well with the period in which the patient is unconscious. This is generally also true for the activity in the delta and theta bands. Various manufacturers offer integrated systems with which the depth of sedation/anaesthesia can be measured. Typically a value is computed by methods that are at least partially undisclosed. One example is the Narcotrend index, which provides an automatic classification of the EEG on a scale ranging from 100 (awake) to 0 (very deep hypnosis, EEG suppression). The algorithm is not fully disclosed.

In order to describe the various levels of sedation, also the Modified Observer's Assessment of Alertness/Sedation (MOAA/S) may be used:

| **Level of sedation** | **MOAA/S score** |
|---|---|
| Fully alert | 5 |
| Mild sedation | 4 |
| Moderate sedation | 2 - 3 |
| Deep sedation | 0 - 1 |
| Loss of consciousness | 0 |

It is possible that a decrease in blood pressure (hypotension), in particular cerebral hypotension, may lead to global brain ischaemia. Hypotension associated with general anaesthesia may cause serious problems including brain damage. Certain groups of patients are particularly at risk, and brain damage is a particular concern in these groups, including infants, children and patients with a cognitive impairment (which is at least mild cognitive impairment). These groups of patients are especially sensitive to hypotension when sedated to a MOAA/S score of ≤ 1 (deep sedation and loss of consciousness).

Without being bound by theory, the higher sensitivity of infants and children might be due to the fact that their developing brains have more plasticity and are less robust against untoward circumstances.

In patients with at least mild cognitive impairment it is often observed that after general anaesthesia the cognitive impairment is exacerbated, and for example pre-dementia progresses to full-blown dementia. It is hypothesised that in the already compromised brains of these patients hypotension causes more damage. Mild cognitive impairment (also called incipient dementia or isolated memory impairment) as used herein means a neurological disorder defined by cognitive impairments beyond those expected from the patient's age and education, but not significant enough to interfere with the patient's daily activities. It mainly occurs in the elderly. Mild cognitive impairment may be a transitional stage between normal ageing and dementia. It may be of the type in which memory loss is the predominant symptom (in which case it is called amnestic mild cognitive impairment and often perceived to be a prodromal stage of Alzheimer's disease), or of the type in which the predominant symptom is in other domains than memory (non-amnestic mild cognitive impairment).

For general anaesthesia inhalational anaesthetics like sevoflurane and intravenous anaesthetics like propofol are available. Propofol is the most widely used intravenous anaesthetic worldwide. It is used in induction and maintenance of general anaesthesia. Although the advantages of propofol are well known and generally accepted (such as the high drug clearance even after long term infusion and the short induction and recovery times), there are known disadvantages. Most importantly, propofol depresses the cardiovascular system by decreasing sympathetic nervous system activity. The main effect is a dose dependent decrease in systemic vascular resistance. This effect is followed by a substantial decrease in blood pressure of about 20 to 40%. This effect may also be potentiated by opioid analgesics. Sodium thiopental, another intravenous anaesthetic, also causes hypotension, although the decrease in blood pressure is usually less severe than with propofol. A blood pressure-lowering effect is also known for sevoflurane, for instance, which has a fast onset and offset of activity and is one of the most commonly used inhalational anaesthetics. Generally, a number of the drugs currently used for general anaesthesia cause hypotension and may thus lead to the above-mentioned problems.

Remimazolam (3-[(4S)-8-bromo-1-methyl-6-(2-pyridinyl)-4H-imidazo[1,2-a][1,4]benzodiazepin-4-yl] propionic methyl ester, also called CNS 7056) is an ultra-short acting benzodiazepine sedative. Induction and recovery times are short. Its proposed indications are in short term procedural sedation in patients 18 years of age or older undergoing diagnostic or therapeutic procedures and in the induction and maintenance of general anaesthesia in patients 18 years of age or older. The safety of remimazolam in combination with an opioid (in particular fentanyl) is disclosed in WO 2012/062439 A1. A general use of remimazolam in the induction and maintenance of general anaesthesia is disclosed in WO 2014/034890 A1.

There is a medical need to perform surgery or other procedures requiring general anaesthesia also on the above-mentioned groups of patients who are particularly at risk of suffering from brain damage. It also needs to be taken into account that a large part of the persons who need to undergo general anaesthesia are elderly people, and that age is correlated with the occurrence of mild cognitive disorder. Moreover, it is generally a desirable goal to minimise or avoid brain damage arising from general anaesthesia. It is the object of the present invention to reduce or avoid the safety concerns present in the prior art when a MOAA/S score of ≤ 1 is to be achieved.

In a first aspect, the present invention relates to a benzodiazepine or pharmaceutically acceptable salt thereof for use in inducing and/or maintaining a MOAA/S score of ≤ 1 in a human patient selected from an infant, a child and a patient with a cognitive impairment that is at least mild cognitive impairment,
wherein the benzodiazepine or salt is defined by having the property of leading to one or more (preferably all) of the following characteristics of an electroencephalogram of a human reference subject in case said benzodiazepine or salt is administered to said reference subject to induce and/or maintain a MOAA/S score of ≤ 1:
(a) the absolute power in a frequency region selected within the range from 0.5 to < 8 Hz reaches its maximum after the first 5 min in which the MOAA/S score is ≤ 1,
(b) after the point in time when a MOAA/S score of ≤ 1 is first reached, the absolute power in a frequency region selected within the range from 0.5 to < 8 Hz reaches its maximum after that in the range from 8 to < 13 Hz, that in the range from 7.5 to 12.5 Hz or that in the range from 13 to < 30 Hz,
(c) the absolute power in a frequency region selected within the range from 0.5 to < 8 Hz is lower than that in the range from 7.5 to 12.5 Hz, that in the range from 8 to < 13 Hz, that in the range of > 12.5 Hz or that in the range from 13 to < 30 Hz during the first 5 min in which the MOAA/S score is ≤ 1,
(d) during the first 5 min in which the MOAA/S score is ≤ 1, the relative power in a frequency region selected within the range from 0.5 to < 8 Hz is not higher than 140% of that immediately before the point in time when a MOAA/S score of ≤ 1 is first reached, wherein the relative power in said frequency region indicates the absolute power in said frequency region in relation to the absolute power summed over the range from 0.5 to < 30 Hz,
(e) when the MOAA/S score returns to a value of > 1, the absolute power in a frequency region selected within the range from 0.5 to < 8 Hz has not yet returned to that immediately before the point in time when a MOAA/S score of ≤ 1 is first reached.

The absolute power of a band is the integral of all of the power values within its frequency range. The relative power of a band indicates the absolute power of this band in relation to the absolute power summed over the all bands considered.

Herein, an infant is defined as a person of less than 1 year of age, and a child is defined as a person of 1 to 12 years of age

A preferred infant is up to 9 months, up to 6 months, up to 4 months, up to 3 months, up to 2 months and most preferably up to 1 month of age.

A preferred child is 1 to 9 years, 1 to 6 years, 1 to 4 years, 1 to 3 years, 1 to 2 years or most preferably 1 year to 1.5 years of age.

A preferred patient with cognitive impairment is at least 65 years, at least 70 years, at least 75 years or most preferably at least 80 years of age.

A preferred cognitive impairment is dementia, more preferably Alzheimer's disease. These types of cognitive impairment are more severe than mild cognitive impairment. Preferred kinds of mild cognitive impairment are non-amnestic mild cognitive impairment and more preferably amnestic mild cognitive impairment.

In a second aspect, the present invention relates to a benzodiazepine or pharmaceutically acceptable salt thereof for use in inducing and/or maintaining a MOAA/S score of ≤ 1 in a patient without causing brain damage,
wherein the benzodiazepine or salt is defined by having the property of leading to one or more of the following characteristics of an electroencephalogram of a human reference subject in case said benzodiazepine or salt is administered to said reference subject to induce and/or maintain a MOAA/S score of ≤ 1:
(a) the absolute power in a frequency region selected within the range from 0.5 to < 8 Hz reaches its maximum after the first 5 min in which the MOAA/S score is ≤ 1,
(b) after the point in time when a MOAA/S score of ≤ 1 is first reached, the absolute power in a frequency region selected within the range from 0.5 to < 8 Hz reaches its maximum after that in the range from 8 to < 13 Hz, that in the range from 7.5 to 12.5 Hz or that in the range from 13 to < 30 Hz,
(c) the absolute power in a frequency region selected within the range from 0.5 to < 8 Hz is lower than that in the range from 7.5 to 12.5 Hz, that in the range from 8 to < 13 Hz, that in the range of > 12.5 Hz or that in the range from 13 to < 30 Hz during the first 5 min in which the MOAA/S score is ≤ 1,
(d) during the first 5 min in which the MOAA/S score is ≤ 1, the relative power in a frequency region selected within the range from 0.5 to < 8 Hz is not higher than 140% of that immediately before the point in time when a MOAA/S score of ≤ 1 is first reached, wherein the relative power in said frequency region indicates the absolute power in said frequency region in relation to the absolute power summed over the range from 0.5 to < 30 Hz,
(e) when the MOAA/S score returns to a value of > 1, the absolute power in a frequency region selected within the range from 0.5 to < 8 Hz has not yet returned to that immediately before the point in time when a MOAA/S score of ≤ 1 is first reached.

In the second aspect of the present invention, a preferred patient is a human.

Generally, a patient may be a male or a female with no preference. However, in one embodiment it may be preferred that the patient is selected from males. In another embodiment it may be preferred that the patient be selected from females.

The above description of the second aspect of the invention uses the term "for use in inducing and/or maintaining a MOAA/S score of ≤ 1 in a patient without causing brain damage", which refers to a specific goal and purpose of the medical use. This term refers to one intention underlying the medical use. Instead of this term, the following terms may alternatively be used:
- "for use in inducing and/or maintaining a MOAA/S score of ≤ 1 in a patient and avoiding brain damage caused thereby",
- "for use in inducing and/or maintaining a MOAA/S score of ≤ 1 in a patient and avoiding associated brain damage",
- "for use in inducing and/or maintaining a MOAA/S score of ≤ 1 in a patient, wherein no brain damage occurs",
- "for use in inducing and/or maintaining a MOAA/S score of ≤ 1 in a patient without causing cerebral ischaemia",
- "for use in inducing and/or maintaining a MOAA/S score of ≤ 1 in a patient and avoiding cerebral ischaemia caused thereby",
- "for use in inducing and/or maintaining a MOAA/S score of ≤ 1 in a patient and avoiding associated cerebral ischaemia",
- "for use in inducing and/or maintaining a MOAA/S score of ≤ 1 in a patient, wherein no cerebral ischaemia occurs",
- "for use in inducing and/or maintaining a MOAA/S score of ≤ 1 in a patient and minimising a dampening effect on the hypothalamus and/or brainstem", and/or
- "for use in inducing and/or maintaining a MOAA/S score of ≤ 1 in a patient and minimising cerebral hypotension".

The present inventors have found that after the administration of remimazolam, human subjects display certain features in their electroencephalogram (EEG). These features may also be referred to as an "EEG signature pattern". More specifically, these features allow direct conclusions about the activity on the brainstem (as explained in the following), which controls inter alia hypotension. As a result, whether or not a specific benzodiazepine leads to such an EEG signature pattern (that is, whether it has the above-mentioned property when administered to the reference subject as described, and optionally the additional property as further defined below) allows conclusions as to whether or not hypotension, in particular cerebral hypotension, can be sufficiently reduced or avoided when deep sedation and/or loss of consciousness is achieved. Thus, a benzodiazepine showing the EEG signature pattern, including remimazolam, will cause exceptionally low cerebral hypotension when used for inducing and/or maintaining a MOAA/S score of ≤ 1.

As mentioned above, anaesthetics often depress the brainstem and thus foster cerebral hypotension, which may have adverse consequences. As opposed to that, the benzodiazepine or salt for use according to the first or second aspect of the present invention allows the achievement of low MOAA/S scores such as those required for general anaesthesia (including loss of consciousness) with adequate safety, because its EEG signature pattern means that it has a reduced depressing activity on the brainstem. Such a benzodiazepine (or salt) is therefore especially suited for administration to the problematic patient groups having a higher risk for damage from cerebral hypotension recited in the description of the first aspect of the present invention. Consequently, these problematic patient groups may be treated adequately. Such a benzodiazepine (or salt) which avoids cerebral hypotension, or limits it to a non-critical extent, allows achieving the goal to treat patients without causing brain damage. Thus, the second aspect of the present invention relates to inducing and/or maintaining a MOAA/S score of ≤ 1 in a patient without causing brain damage. This way, concerns present in the prior art are eliminated or at least reduced.

Preferably, the above-mentioned property by which the benzodiazepine or salt is defined includes the above characteristics (a) to (e) as follows:
- characteristic (a), characteristic (b), characteristic (c), characteristic (d) or characteristic (e),
- characteristics (a) and (b), characteristics (a) and (c), characteristics (a) and (d), characteristics (a) and (e), characteristics (b) and (c), characteristics (b) and (d), characteristics (b) and (e), characteristics (c) and (d), characteristics (c) and (e) or characteristics (d) and (e),
- characteristics (a), (b) and (c), characteristics (a), (b) and (d), characteristics (a), (b) and (e), characteristics (a), (c) and (d), characteristics (a), (c) and (e) or characteristics (a), (d) and (e), characteristics (b), (c) and (d), characteristics (b), (c) and (e), characteristics (b), (d) and (e), characteristics (c), (d) and (e),
- characteristics (a), (b), (c) and (d), characteristics (a), (b), (c) and (e), characteristics (a), (b), (d) and (e), characteristics (a), (c), (d) and (e) or characteristics (b), (c), (d) and (e),
   or
- characteristics (a), (b), (c), (d) and (e).

The less depressing activity there is on the brainstem, the less hypotension (including cerebral hypotension) the patient develops. It is known that an increased EEG activity in the above-mentioned range from 0.5 to < 8 Hz, in particular in the theta band, is indicative of an (enhanced) inhibiting effect of the hypothalamus on the brainstem. This inhibiting effect is thought to cause a depressing activity on the regions in the brainstem controlling cardiovascular and respiratory functions. Thus such an increased EEG activity is in particular indicative of cardiovascular and respiratory depression. This means that the higher the EEG signals in this range, the more depression a drug elicits on the brainstem, and the more hypotension the drugs causes. A benzodiazepine having the EEG signature pattern shows rather low signals in the theta (and delta) band (that is, in the range from 0.5 to < 8 Hz). As opposed to a benzodiazepine not having this pattern, there will be less cardiovascular depression.

Another useful feature of a benzodiazepine having the EEG signature pattern (as opposed to one not having it) is that due to the reduced or avoided depressing activity on the brainstem there will be less respiratory depression, or none at all.

Such a benzodiazepine also has a delayed effect on the brainstem. An important feature of the EEG signature pattern is the delayed occurrence of signals in the theta and delta bands (in the range from 0.5 to < 8 Hz) after the point in time when a MOAA/S score of ≤ 1 is first reached (or after LOC). This delayed occurrence is in particular exemplified by the above characteristics (a), (b), (c) and (d). Characteristic (a) defines the time when signals in this range reach their maximum in absolute terms, characteristic (b) in relative terms as compared to other bands. Characteristic (c) defines the signal strength in this region as compared to other bands. Characteristic (d) defines the signal strength in this region as compared to that time before the point in time when a MOAA/S score of ≤ 1 is first reached (or before LOC). This delayed occurrence may be interpreted as less depression in the brainstem during the most vulnerable phase of general anaesthesia, namely the induction phase. The delayed occurrence of signals in the delta band is paradoxical to MOAA/S ≤ 1.

A lower activity in the theta and delta bands is also important during a later phase of general anaesthesia, such as the maintenance phase. In general, the lower the signals in the theta band (and the delta band), the more beneficial the effect of the benzodiazepine is with regard to inducing and/or maintaining a MOAA/S score of ≤ 1 in an infant, a child or a patient with at least mild cognitive impairment, and/or without causing brain damage. In the further course of sedation, it may be assumed that (with unimpaired circulatory reflexes) a gradual decrease in peripheral resistance, for which the delayed activity increase in the delta band is an indication, can be compensated by an increased cardiac output due to an increased heart rate, and thus occurs without any relevant drop in blood pressure.

The course of the activities in the delta, theta, beta and gamma bands may be associated to a rather unspecific interaction between the respective benzodiazepine and α subunits of the GABA_{A} receptor. It may be related to the concentration in the blood plasma over time and to the degree of occupancy and allosteric conformation change of the GABA_{A} receptor. Typically, shortly after starting the infusion of the respective benzodiazepine, the activity in the beta and gamma bands increases disproportionately as compared to the activity in the other bands. As the concentration of the benzodiazepine in the blood plasma increases further, the cortical beta and gamma activity decreases again, and the activity of neuron populations in the deeper CNS structures becomes more and more prominent, which is shown by the increase in delta and theta activity (low-frequency, high-amplitude EEG waves). Additionally, the alpha activity moves from occipital to frontal. In the EEG signature pattern, the delayed occurrence of signals in the theta and delta bands, or the reduction in activity in the theta and delta bands, may be interpreted as a benzodiazepine-induced inhibition of activity of (sub)cortical neuron populations.

Characteristic (e) describes that the delay observed in the range from 0.5 to < 8 Hz it is also observed after the administration of the benzodiazepine or salt has already stopped and the patient slowly regains consciousness.

Preferred embodiments of the above characteristics may be described as follows:
(a) the absolute power in a frequency region selected within the range from 0.5 to < 8 Hz reaches its maximum after the first 10 min, after the first 15 min, after the first 20 min, after the first 25 min or most preferably after the first 30 min in which the MOAA/S score is ≤ 1,
(b) after the point in time when a MOAA/S score of ≤ 1 is first reached, the absolute power in a frequency region selected within the range from 0.5 to < 8 Hz reaches its maximum at least 5 min, at least 10 min, at least 15 min, at least 20 min or most preferably at least 25 min after that in the range from 8 to < 13 Hz, that in the range from 7.5 to 12.5 Hz or that in the range from 13 to < 30 Hz,
(c) the absolute power in a frequency region selected within the range from 0.5 to < 8 Hz is lower than that in the range from 7.5 to 12.5 Hz, that in the range from 8 to < 13 Hz, that in the range of > 12.5 Hz or that in the range from 13 to < 30 Hz during the first 10 min, during the first 15 min, during the first 20 min, during the first 25 min, during the first 30 min or most preferably during the first 35 min in which the MOAA/S score is ≤ 1,
(d) during the first 10 min in which the MOAA/S score is ≤ 1, the relative power in a frequency region selected within the range from 0.5 to < 8 Hz is not higher than 130%, not higher than 120%, not higher than 110%, or more preferably not higher than 100% of that immediately before the point in time when a MOAA/S score of ≤ 1 is first reached,
   during the first 15 min in which the MOAA/S score is ≤ 1, the relative power in a frequency region selected within the range from 0.5 to < 8 Hz is not higher than 130%, not higher than 120%, not higher than 110%, or more preferably not higher than 100% of that immediately before the point in time when a MOAA/S score of ≤ 1 is first reached, or most preferably
   during the first 20 min in which the MOAA/S score is ≤ 1, the relative power in a frequency region selected within the range from 0.5 to < 8 Hz is not higher than 130%, not higher than 120%, not higher than 110%, or more preferably not higher than 100% of that immediately before the point in time when a MOAA/S score of ≤ 1 is first reached,
   wherein the relative power in said frequency region indicates the absolute power in said frequency region in relation to the absolute power summed over the range from 0.5 to < 30 Hz,
(e) the absolute power in a frequency region selected within the range from 0.5 to < 8 Hz returns to that immediately before the point in time when a MOAA/S score of ≤ 1 is first reached no earlier than 5 min, or most preferably no earlier than 10 min after the point in time when the MOAA/S score returns to a value of > 1.

Preferably, in characteristic (a), (b), (c), (d) and (f) the absolute power is a time-average over 10 s, 20 s, 30 s, 40 s, 50 s or most preferably 1 min (or in some instances even above 1 min, such as 1.25 min, 1.5 min, 1.75 min or 2 min) and in characteristic (e) the relative power is a time-average over 10 s, 20 s, 30 s, 40 s, 50 s or most preferably 1 min (or in some instances even above 1 min, such as 1.25 min, 1.5 min, 1.75 min or 2 min).

In a preferred embodiment the frequency region selected within the range from 0.5 to < 8 Hz is 0.5 to 3.5 Hz, 3.5 to 7.5 Hz, 0.5 to < 2 Hz, 0.5 to 2 Hz, 2 to < 5 Hz, 2 to 5 Hz (low theta subband), 5 to < 8 Hz (high theta subband), 2 to < 8 Hz, 0.5 to < 3 Hz or 0.5 to 5 Hz.

Generally, the above human reference subject is different from to the above patient. However, in one embodiment, the reference subject and the patient are identical.

Preferably. the benzodiazepine or salt for use according to the invention (first and/or second aspect) is defined by having the above-mentioned property in case said benzodiazepine or salt is administered to said reference subject under one or more of the following conditions:
(I) continuous intravenous administration of the benzodiazepine or salt,
(II) administration of the benzodiazepine or salt at a rate of 5 mg/min for 5 min, followed by a rate of 3 mg/min for 15 min and of 1 mg / min for another 15 min,
(III) electrode placement on the reference subject's scalp according to the international 10/20 system; frontal lead F3-Cz and Fp1 as ground,
(IV) recording the electroencephalogram with a Nicolet v32 amplifier and NeuroWorks 8 software (Natus Europe, Planegg, Germany),
(V) continuous recording of the electroencephalogram, digitisation at a sampling rate of 500 Hz with a gain of 0.1525902 µV/unit, first low-pass filtered via a finite impulse response FIR at 47 Hz, down sampled to 125 Hz, and subsequently high-pass filtered with a FIR filter at 0.5 Hz and segmented into EEG epochs of 8.192 s (2¹⁰ data points) length,
(VI) placement of three EEG electrodes on the reference subject's forehead with a distance of at least 8 cm between the two signal electrodes,
(VII) recording the electroencephalogram with a Narcotrend-Compact M monitor,
(VIII) reference subject's age of 20 to 38 years, preferably of 21 to 29 years,
(IX) reference subject's body weight of 64 to 99 kg, preferably of 67 to 87 kg,
(X) reference subject's body mass index of 21 to 29 kg/m², preferably of 22 to 26 kg/m²,
(XI) reference subject's height of 169 to 197 cm, preferably of 171 to 187 cm,
(XII) reference subject is a male.

Regarding these conditions, a combination of the above conditions (I) and (II), a combination of the above conditions (III) and (IV), a combination of the above conditions (III), (IV) and (V), a combination of the above conditions (IV) and (V), a combination of the above conditions (VI) and (VII), a combination of the above conditions (VIII), (IX), (X) and (XI), a combination of the above conditions (VIII), (IX), (X), (XI) and (XII), and a combination of the above conditions (IX) and (X) are preferred. More preferred are a combination of the above conditions (I), (II), (III), (IV), (V), (VIII), (IX), (X), (XI) and (XII), or a combination of the above conditions (I), (II), (VI), (VII), (VIII), (IX), (X), (XI) and (XII) is most preferred.

Concerning condition (II), all doses refer to the weight of the free base.

Preferably, the benzodiazepine or salt for use according to the invention is further defined by having the additional property of leading to the following characteristic of the electroencephalogram of said reference subject (preferably when administered under the conditions just described): during the first 5 min (preferably during the first 10 min) in which the MOAA/S score is ≤ 1, the absolute power in the range from 13 to < 30 Hz, in the range of > 12.5 Hz, or in the range from 30 to 50 Hz is not lower than that immediately before the point in time when a MOAA/S score of ≤ 1 is first reached.

The use is in inducing and/or maintaining a MOAA/S score of ≤ 1 in the patient. Preferably, the use is in inducing a MOAA/S score of ≤ 1 in the patient, more preferably in inducing, but not maintaining a MOAA/S score of ≤ 1 in the patient. Other uses are in inducing and maintaining a MOAA/S score of ≤ 1 in the patient, in maintaining a MOAA/S score of ≤ 1 in the patient, and in maintaining, but not inducing a MOAA/S score of ≤ 1 in the patient. Preferably, these MOAA/S scores occur in the context of general anaesthesia.

The MOAA/S score of ≤ 1 is preferably a MOAA/S score of 0 to 1, more preferably of < 1, yet more preferably of 0 to less than 1 and most preferably of 0.

A preferred embodiment relates to the benzodiazepine or salt for use according to the invention, wherein said use involves
- administration to the patient by intravenous infusion (preferably continuous intravenous infusion) or
- intrapulmonary and/or intranasal administration to the patient.

Such a continuous intravenous infusion preferably comprises administering a first dose for a first time, then administering a second dose for a second time and optionally a third dose for a third time, wherein the second dose is less than the first dose and the third dose, if applicable, is less than the second dose. One example is about 5 mg/min for about 5 min, then about 3 mg/min for about 15 min, then about 1 mg/min for about 15 min.

Preferred ways of intrapulmonary and intranasal administration are disclosed in WO 2017/178663 A1 and incorporated herein by reference.

A preferred benzodiazepine is according to formula (I) 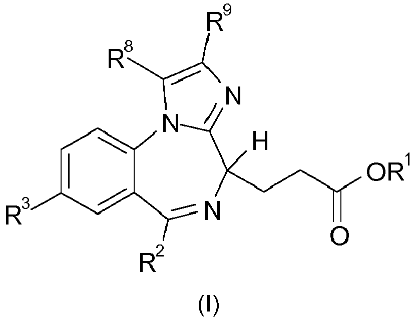 wherein
R¹ is CH₃, CH₂CH₃, CH₂CH₂CH₃, CH(CH₃)₂ or CH₂CH(CH₃)₂;
R² is 2-fluorophenyl, 2-chlorophenyl or 2-pyridyl;
R³ is Cl or Br;
R⁸ is hydrogen, C₁₋₄ alkyl or C₁₋₃ hydroxyalkyl; and
R⁹ is H, C₁₋₄ alkyl, C₁₋₃ hydroxyalkyl or C₁₋₄ alkoxy.

Formula (I) includes benzodiazepines structurally similar to remimazolam. Consequently it may be assumed that after the administration of a benzodiazepine of formula (I), human subjects will display similar features in their electroencephalogram (EEG) and in particular the EEG signature. Consequently it may be assumed that a benzodiazepine of formula (I) will cause exceptionally low cerebral hypotension when used for inducing and/or maintaining a MOAA/S score of ≤ 1.

A particularly preferred benzodiazepine is methyl 3-[(4S)-8-bromo-1-methyl-6-(pyridin-2-yl)-4H-imidazo[1,2-a][1,4]benzodiazepin-4-yl]propanoate (remimazolam). However, in some embodiments it may also be preferable that the benzodiazepine is different from remimazolam.

Another preferred benzodiazepine is lorazepam ((RS)-7-chloro-5-(2-chlorphenyl)-3-hydroxy-2,3-dihydro-1H-1,4-benzodiazepin-2-one).

Concerning dosing, preferably the use involves administration to the patient at a dose of 0.3 to 40 mg/h/kg, wherein the dose refers to remimazolam. A two-step administration using different doses for induction and maintenance is preferred (for example induction 3 to 40 mg/h/kg and/or maintenance 0.3 to 2.5 mg/h/kg).Particularly preferred dosing regimens for remimazolam are disclosed in WO 2014/034890 A1 and incorporated herein by reference.

Another preferred embodiment is administration to the patient at a fixed dose irrespective of the patient's body weight. Particularly preferred fixed dose regimens for remimazolam are disclosed in WO 2012/062439 A1 and incorporated herein by reference.

Preferably, for benzodiazepines other than remimazolam, equivalent doses based on the sedative effect (preferably according to the MOAA/S score) are used and converted from the remimazolam dose accordingly.

A preferred salt of the benzodiazepine for use according to the invention is selected from the group consisting of halogenides (preferably fluoride, chloride or bromide), sulfate, organic sulfates (preferably aromatic sulfates), sulfonate, organic sulfonates (preferably aromatic sulfonates, such as toluene sulfonate and in particular benzene sulfonate), nitrate, phosphate, salicylate, tartrate, citrate, maleate, formiate, malonate, succinate, isethionate, lactobionate and sulfamate.

Preferably, the benzodiazepine or salt for use according to the invention is used together with a further active ingredient or a pharmaceutically acceptable excipient. Excipients include carriers, diluents, buffers and vehicles.

A preferred further active ingredient is an opioid. Particularly preferred opioids, such as fentanyl and its derivatives, and their dosing, are disclosed in WO 2012/062439 A1 and incorporated herein by reference.

A preferred excipient is a disaccharide and/or a dextran. Particularly preferred disaccharides (such as lactose, maltose, sucrose and trehalose, in particular lactose) and dextrans (such as those with a molecular weight of less than 150 kD) and their amounts and combinations are disclosed in WO 2013/174883 A1 and incorporated herein by reference.

### Examples

### Example 1: EEG measurements after remimazolam administration

Subjects (20 healthy adult males) were administered remimazolam as a continuous intravenous infusion of 5 mg/min for 5 min, 3 mg/min for the next 15 min, and 1 mg/min for further 15 min. The total administered amount of CNS7056 was 85 mg in each subject. The demographic data was, given as mean ± SD (range):

| | |
|---|---|
| Age (years) | 25.1 ± 4.3 (20 to 38) |
| Weight (kg) | 77.2 ± 9.6 (64 to 99) |
| Height (cm) | 179 ± 8 (169 to 197) |
| Body mass index (kg/m²) | 23.9 ± 1.8 (20.8 to 28.7) |

The EEG was recorded with a Nicolet v32 amplifier and NeuroWorks 8 software (Natus Europe, Planegg, Germany) starting ca. 60 min before start of infusion until 90 min after stop of infusion. The electrodes were placed on the subjects' scalps according to the international 10/20 system at C3, C4, F3, F4, O1, O2, Cz (reference), and Fp1 (ground). It was found that (unlike in the case of e.g. midazolam) F3-Cz and F4-Cz are the most suitable positions, as the EEG parameters derived therefrom showed the highest correlation with the clinical sedation score (MOAA/S) and had the highest signal to noise ratio. For analysis, the EEG was digitised with a sampling rate of 500 Hz and a gain of 0.1525902 µV/unit. The digitised EEG was first low-pass filtered via a finite impulse response FIR at 47 Hz, then down sampled to 125 Hz, and subsequently high-pass filtered with a FIR filter at 0.5 Hz. Power spectrum analysis (Fast Fourier Transform, FFT) was performed on EEG epochs of 8.192 s (1024 data points) length.

Additional EEG recording was performed using the Narcotrend®-Compact M monitor version 3.0 (MT MonitorTechnik, Bad Bramstedt, Germany), starting ca. 60 min before start of infusion until 90 min after stop of infusion. For this purpose, three EEG electrodes were placed on the subject's forehead with a distance of at least 8 cm between the two signal electrodes. The Narcotrend index (NCT) as well as absolute and relative power in the range of 0.5 to 3.5 Hz (in this device referred to as delta band), in the range of 3.5 to 7.5 Hz (in this device referred to as theta band), in the range of 7.5 to 12.5 Hz (in this device referred to as alpha band) in the range of 12.5 to 45 Hz (in this device referred to as beta band) were obtained from the Narcotrend monitor.

The depth of sedation was recorded every two minutes by using the MOAA/S score, from 2 min before start of infusion until three subsequent MOAA/S scores of 5 (alert) were recorded after stop of remimazolam administration.

Additionally the arterial plasma concentrations of remimazolam were measured.

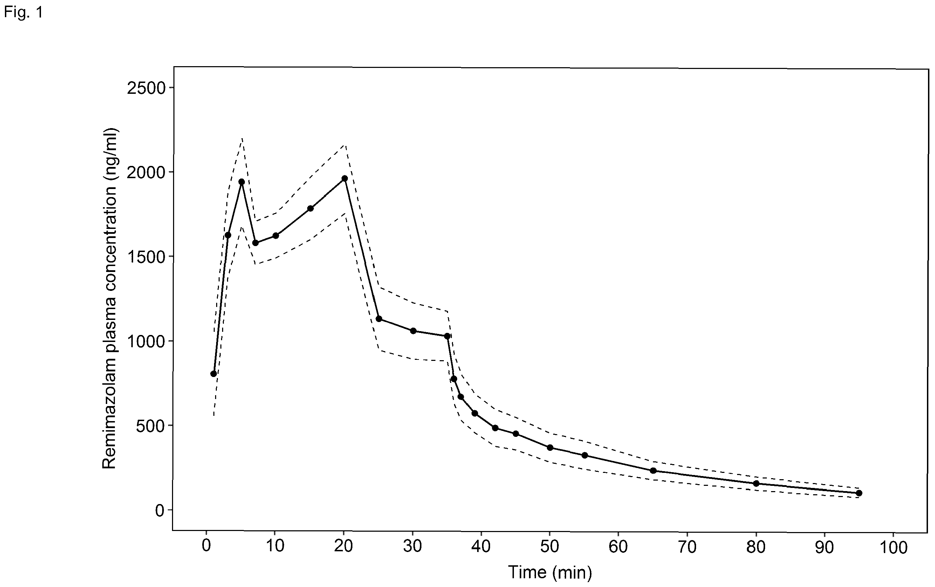

Figure 1 shows the measured plasma concentrations of remimazolam (mean ± SD) from start of infusion (t = 0 min) until t = 95 min, which is 60 min after stop of infusion (N = 20).

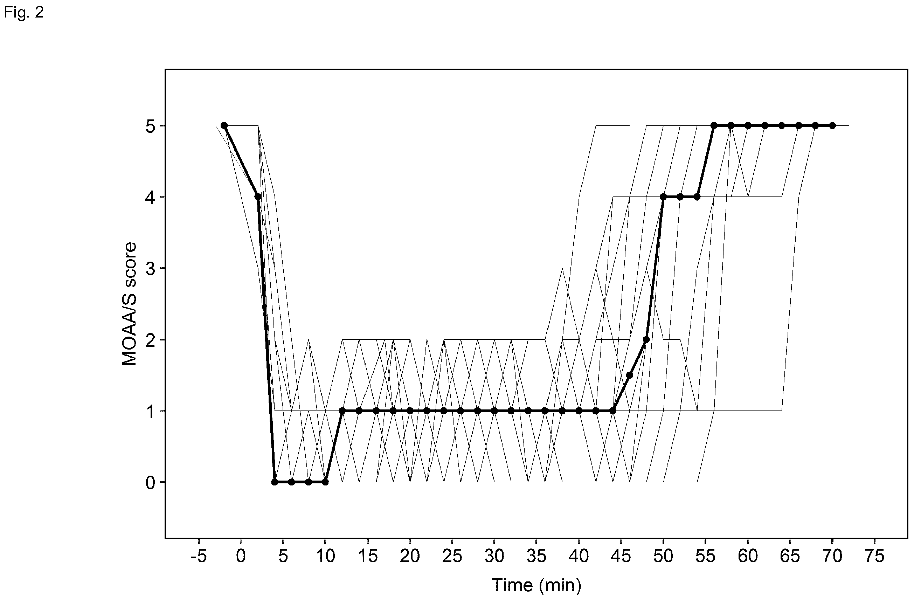

Figure 2 shows the time course of the observed MOAA/S scores (individual data and median). Each thin line shows the individual values of one subject. The bold line shows the median values in the study population (N = 20). The MOAA/S score decreased from a baseline of 5 to values ≤1 within 4.6 ± 1.1 min after start of remimazolam infusion. After that, during the remimazolam infusion, the median MOAA/S score stayed at ≤ 1 (individual values between 0 and 2). After stop of infusion (at 35 min after start of infusion), a MOAA/S score >1 was regained 45.9 ± 7.9 min after start of infusion, and a MOAA/S score of 5 (alert) was regained 54.4 ± 6.7 min after start of infusion.

Taken together, in the EEG recorded from frontal, central and occipital electrodes, in particular in the frontal EEG (F3-Cz and F4-Cz) recorded with the Nicolet system, delayed theta and delta bands were observed at the beginning and after the end of the infusion. The EEG was further characterised by an activity increase in the alpha band during the first 20 minutes and also in the beta band during the first 10 minutes of the infusion. In the further course, EEG activity in the delta band and also in the range from 0.5 to 3.5 Hz increased.

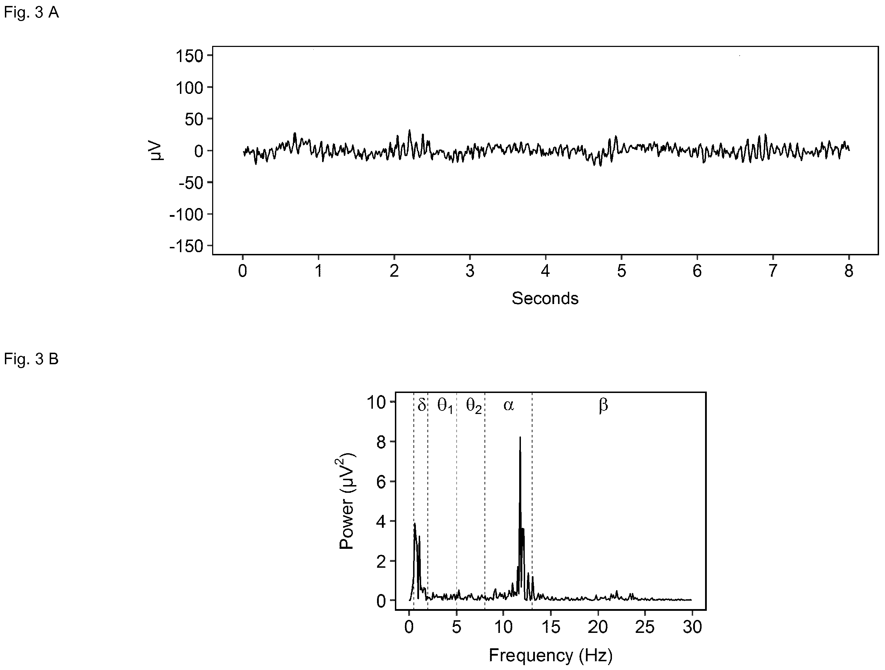

Figures 3 to 12 show the data obtained with the Nicolet system.

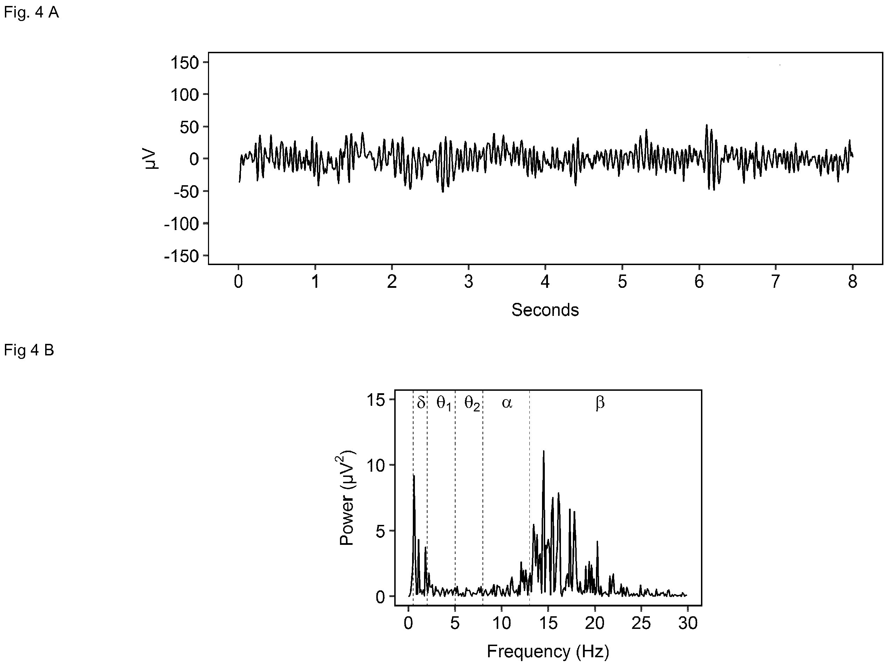

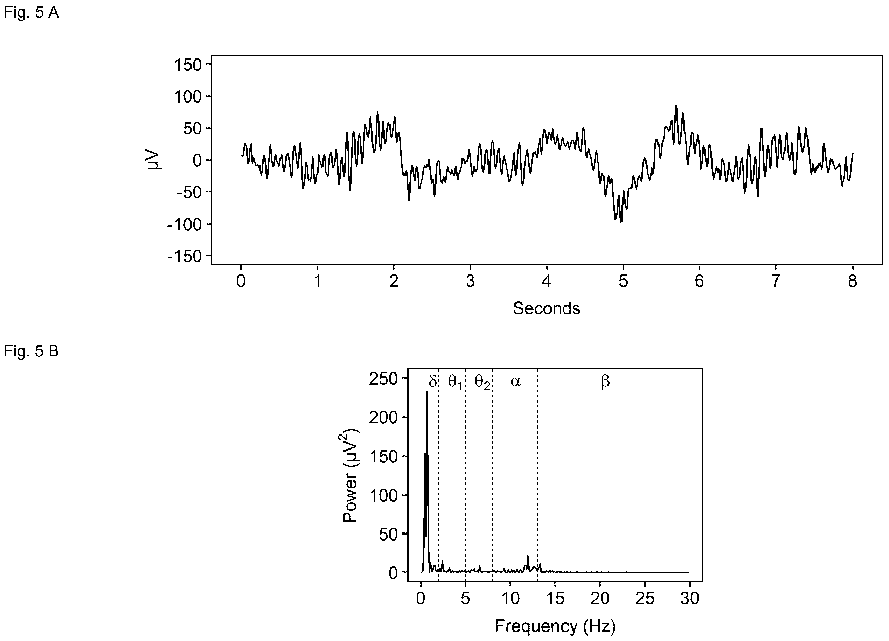

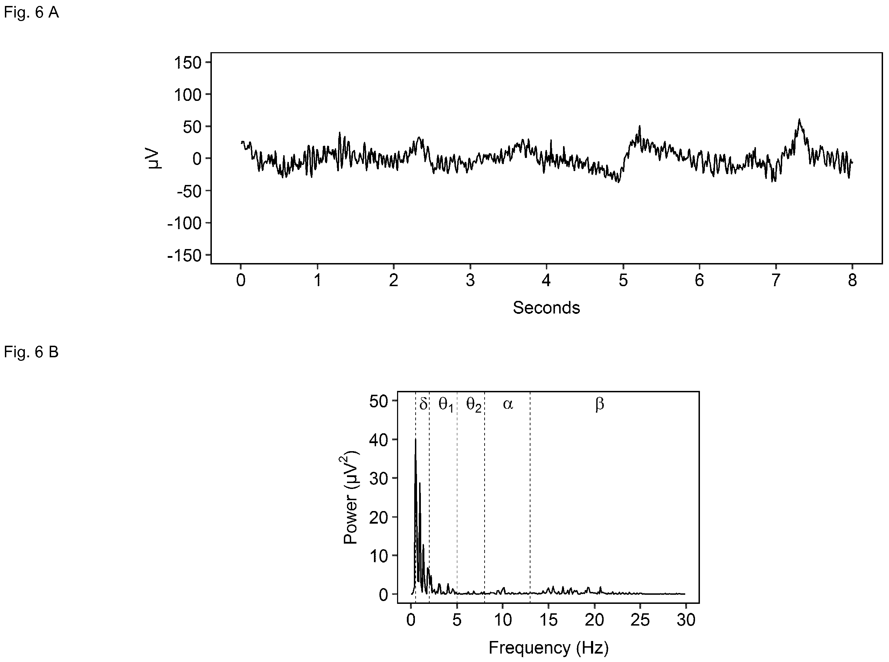

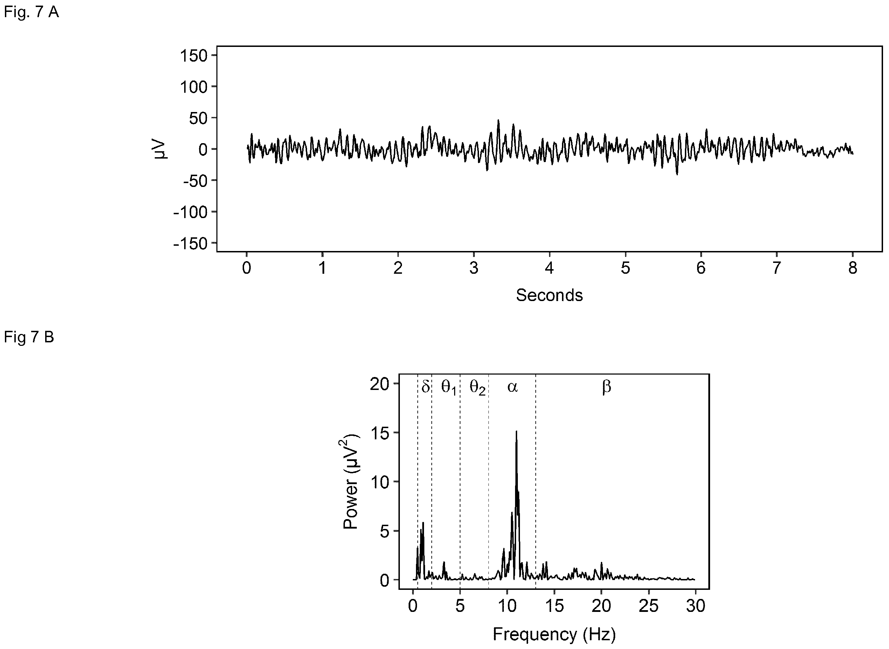

In Figures 3 to 7, typical EEG patterns obtained from the frontal lead F3 (i.e., F3-Cz) of one subject at different time points (Figures 3A, 4A, 5A, 6A and 7A) together with the corresponding power spectra (Figures 3B, 4B, 5B, 6B and 7B) are shown. In this paragraph and in Figures 3 to 7, the following band designations are used, which are specific for the Nicolet device: 0.5 to 2 Hz (delta, δ), 2 to 5 Hz (theta₁, θ₁), 5 to 8 Hz (theta₂, θ₂), 8 to 13 Hz (alpha, α), 13 to 30 Hz (beta, β). At baseline (4.9 min before start of infusion, remimazolam concentration: 0 ng/ml, MOAA/S score of 5), the EEG was characterised by high activity in the alpha band (Fig. 3A, 3B). 4.5 min after start of the infusion, the MOAA/S score was 1, the remimazolam concentration was 1548 ng/ml, and the EEG was characterised by high activity in the beta band (Fig. 4A, 4B). Immediately after stop of infusion (35.6 min after start of the infusion), the MOAA/S score was still 1 (remimazolam concentration: 945 ng/ml), whereas the EEG was now characterised by high activity in the delta band (Fig. 5A, 5B). When the subject regained alertness (66.2 min after start of infusion, remimazolam concentration: 270 ng/ml, MOAA/S score of 5), the EEG was characterised by high activity in the delta band in combination with some activity in the beta band (Fig. 6A, 6B). At the end of the EEG recording (89.7 min after start of infusion, remimazolam concentration: 141 ng/ml, MOAA/S score of 5), the EEG was again characterised by high activity in the alpha band (Fig. 7A, 7B).

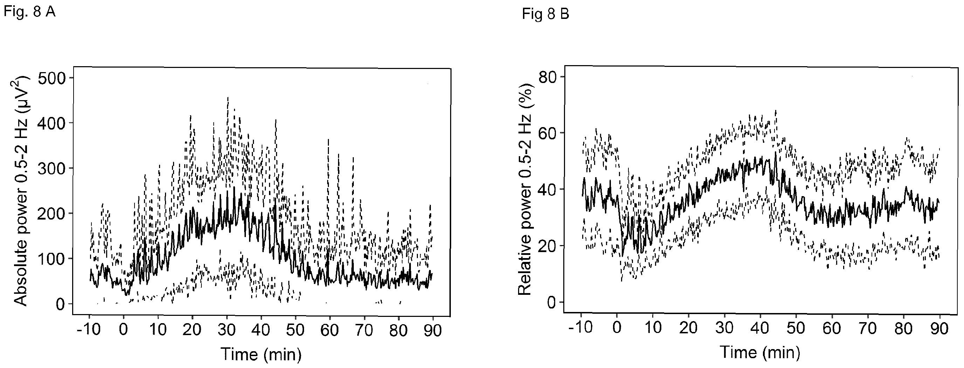

Figures 8 to 12 show the time courses (mean ± SD) of absolute power (Figures 8A, 9A, 10A, 11A and 12A) and relative power (Figures 8B, 9B, 10B, 11B and 12B) in different frequency regions of the EEG recorded from frontal lead F3 (i.e., F3-Cz) with the above Nicolet system (N = 20). The band designations are the same as described above for Figures 3 to 7.

Considering the mean values, the absolute power in the range of 0.5 to 2 Hz (Fig. 8A) reached its maximum at about 35 min after the start of the infusion (after about the first 30 min in which the MOAA/S score was ≤ 1). The absolute power in the range of 2 to 5 Hz (Fig. 9A) and that in the range of 5 to 8 Hz (Fig. 10A) reached its maximum at about 20 min after the start of the infusion (after the first 15 min in which the MOAA/S score was ≤ 1)

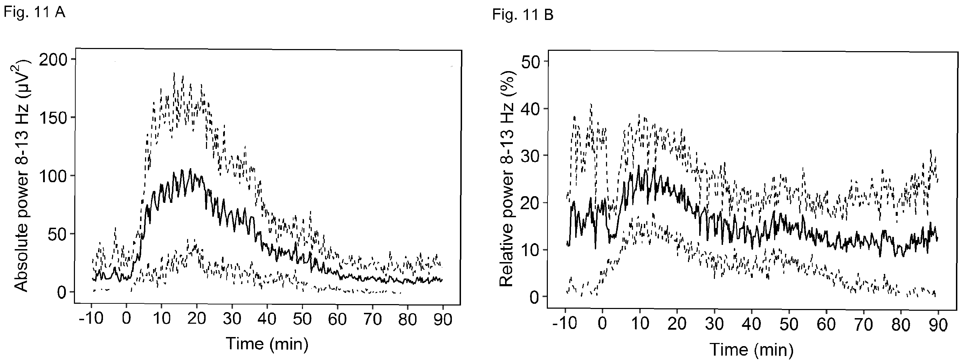

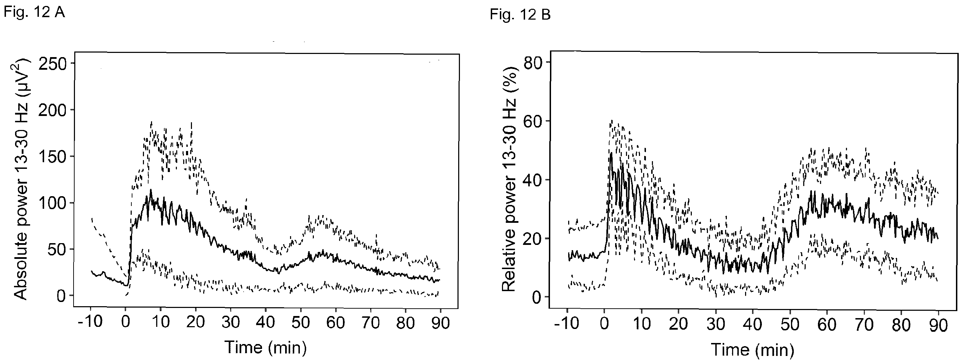

The absolute power in the range of 8 to 13 Hz (Fig. 11A) reached its maximum at about 15 min after the start of the infusion, and that in the range of 13 to 30 Hz (Fig. 12A) reached its maximum at about 5 min after the start of the infusion.

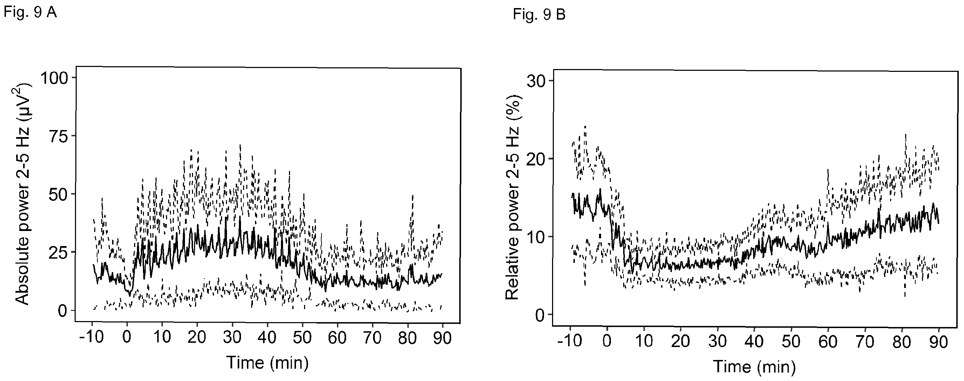

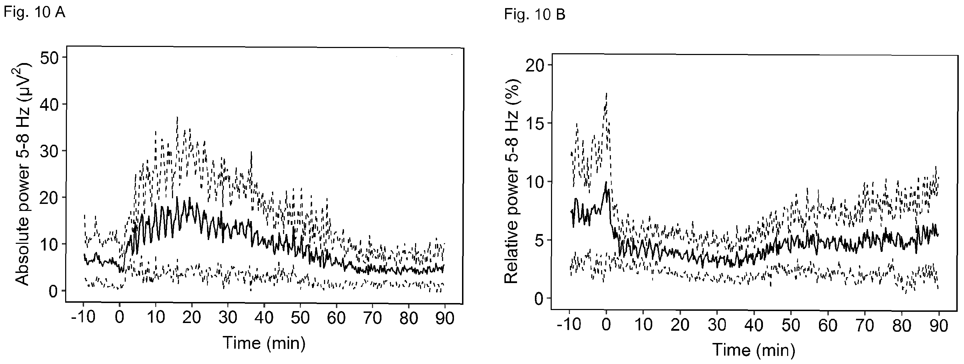

During the first 5 min in which the MOAA/S score was ≤ 1 (from about 5 to 10 min after the start of the infusion) the absolute power in the range of 2 to 5 Hz (Fig. 9A) was not higher than 30 µV², and that in the range of 5 to 8 Hz (Fig. 10A) was below 20 µV². Within this time, the absolute power in the range of 8 to 13 Hz (Fig. 11A) was not lower than about 50 µV², and that in the range of 13 to 30 Hz (Fig. 12A) was not lower than about 75 µV². The absolute power in the range of 2 to 5 Hz (Fig. 9A) remained at values not higher than about 40 µV² throughout the observation period, and that in the range of 8 to 13 Hz, as well as that in the range of 13 to 30 Hz (Fig. 11A and 12A, respectively), was not lower than about 40 µV² during at least the first 30 min in which the MOAA/S score was ≤ 1 (until at least 35 min after the start of the infusion). The absolute power in the range of 5 to 8 Hz (Fig. 10A) remained below 20 µV² throughout the observation period, and still after the first 35 min in which the MOAA/S score was ≤ 1 (40 min after the start of the infusion) it was lower than that in the range of 8 to 13 Hz, as well as that in the range of 13 to 30 Hz (Fig. 11A and 12A, respectively).

When the MOAA/S score returned to a value of > 1 (about 45 min after the start of infusion), the absolute power in the range of 0.5 to 2 Hz (Fig. 8A) fluctuated around values that were about twice as high as those immediately before the point in time when a MOAA/S score of ≤ 1 was first reached (about 5 min after the start of infusion). The absolute power in the range of 2 to 5 Hz (Fig. 9A) also fluctuated around values higher than those immediately before the point in time when a MOAA/S score of ≤ 1 was first reached.

The corresponding time courses of relative power are shown in Figures 8B, 9B, 10B, 11B and 12B. During the first 5 min in which the MOAA/S score was ≤ 1 (from about 5 to 10 min after the start of the infusion), the relative power in the range of 0.5 to 2 Hz (Fig. 8B) fluctuated around the same values as immediately before the point in time when a MOAA/S score of ≤ 1 was first reached (about 5 min after the start of infusion).

During the first 5 min in which the MOAA/S score was ≤ 1, the relative power in the range of 2 to 5 Hz (Fig. 9B) was not higher than about 8%, and the relative power in the range of 5 to 8 Hz (fig. 4H) was below 5%, which was in each case about the same value as immediately before the point in time when a MOAA/S score of ≤ 1 was first reached. This behaviour in the range of 2 to 5 Hz was observed at least during the first 15 min in which the MOAA/S score was ≤ 1, and also after that it increased only minimally; only after more than 30 min in which the MOAA/S score was ≤ 1 there was some more pronounced, but still slight increase. The absolute power in the range of 5 to 8 Hz even decreased until more than30 min in which the MOAA/S score was ≤ 1, and then there was a slight increase also in this frequency range.

It can be seen that in the Nicolet system there was a correlation between the theta band and the delta band: In the first 5 min after reaching LOC (or after the point in time when a MOAA/S score of ≤ 1 was first reached), the increase (if at all) was relatively moderate. Activities in theta and delta band were still increased when the MOAA/S score returned to a value of > 1.

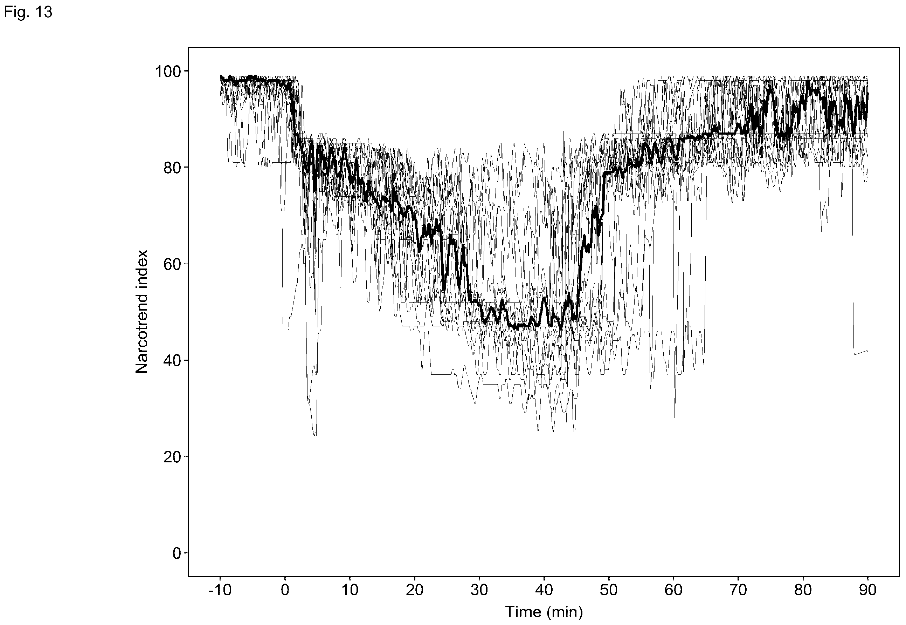

The data from the Narcotrend monitor are summarised in Figures 13 to 18.

The individual time courses and the median time course of the Narcotrend index (NCT) are shown in Figure 13. Each thin line shows the individual values of one particular subject. The bold line shows the median values in the study population (N = 20). Generally, the NCT was characterised by a large interindividual variation. The NCT had an initial value of 96 ± 4, fell to 75 ± 7 after 15 minutes and reached a minimum of 51 ± 15 two minutes after the end of the infusion.

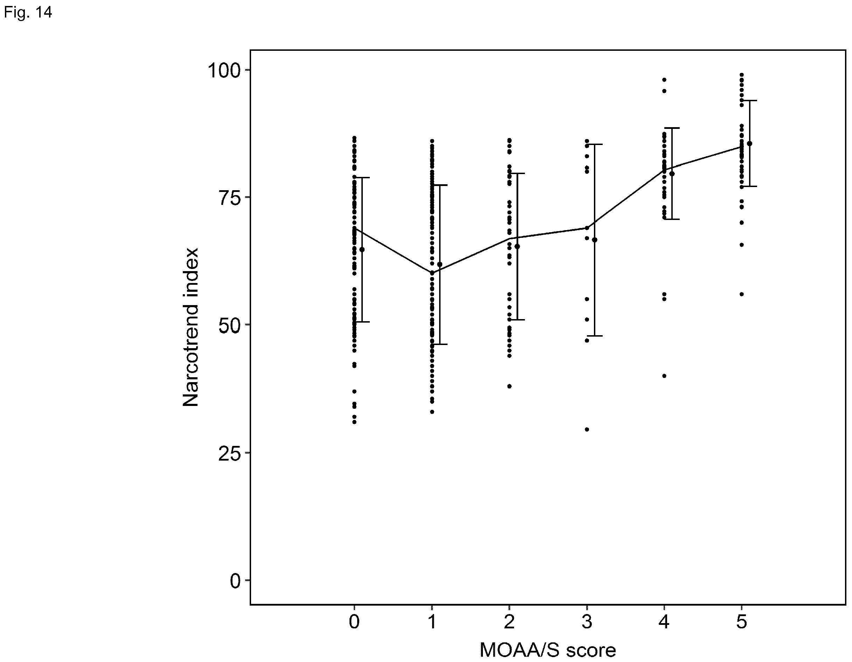

Figure 14 shows the correlation between the MOAA/S score and the Narcotrend index in the study population (N = 20). Individual data are plotted as dots, mean ± SD is plotted as dots with error bars. It can be seen that when a MOAA/S score of 4 or 5 was determined, the Narcotrend indices did not show much variation, unlike when MOAA/S scores of 0 to 3 were determined. Further, the NCT did not decrease monotonously with decreasing MOAA/S score (increasing level of sedation), but was higher for a MOAA/S score of 0 than for a MOAA/S score of 1. This resulted in a relatively weak correlation coefficient R² = 0.24.

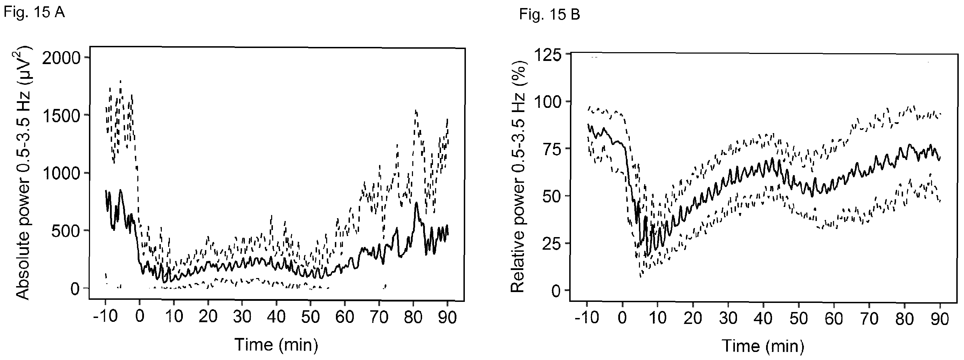

Figures 15 to 18 correspond to Figures 8 to 12, showing the absolute power (Figures 15A, 16A, 17A and 18A) and relative power (Figures 15B, 16B, 17B and 18B) in the frequency bands, which were slightly differently defined for the Narcotrend system than for the Nicolet system. However, the general trends described above were similar for the EEG from the Nicolet system and from the Narcotrend.

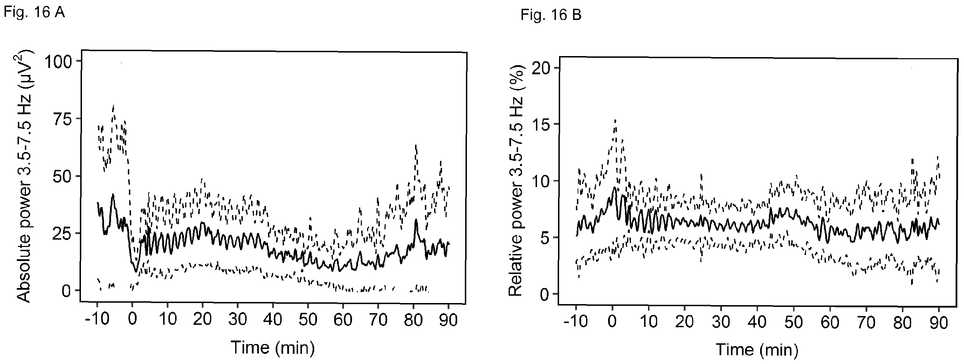

Considering the mean values, it was found that the absolute power in the range of 0.5 to 3.5 Hz (Figure 15A) reached its maximum at about 35 min after the start of the infusion (after the first 30 min in which the MOAA/S score was ≤ 1). The absolute power in the range of 3.5 to 7.5 Hz (Figure 16A) reached its maximum at about 20 min after the start of the infusion (after the first 15 min in which the MOAA/S score was ≤ 1). It was thus found that the absolute power in a frequency region selected within the range from 0.5 to < 8 Hz reached its maximum after the first 5 min in which the MOAA/S score was ≤ 1 (that is after about 10 min after the start of the infusion).

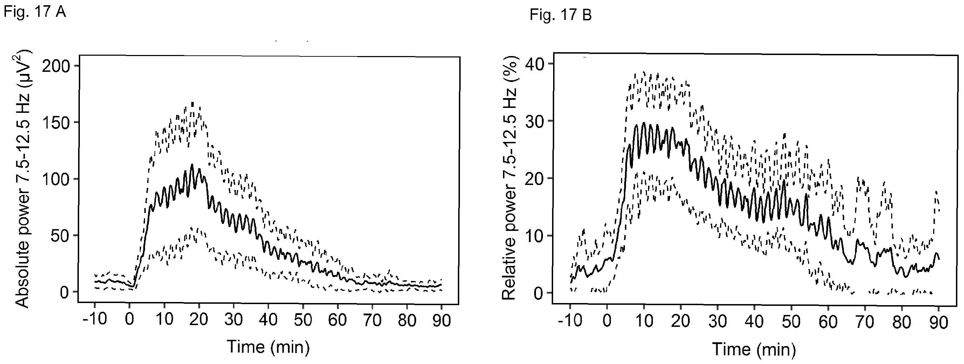

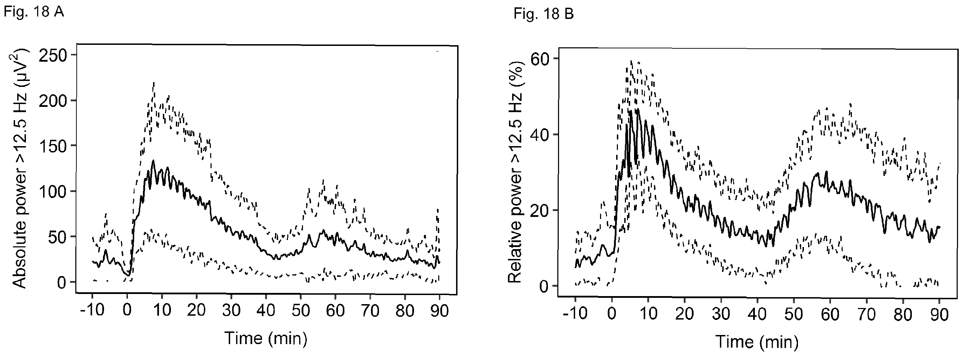

The absolute power in the range of 7.5 to 12.5 Hz (Figure 17A) reached its maximum at about 20 min after the start of the infusion and that in the range of > 12.5 Hz (Figure 18A) at about 10 min after the start of the infusion. Thus, it can be seen that after the point in time when a MOAA/S score of ≤ 1 was first reached (at about 5 min after the start of the infusion), the absolute power in a frequency region selected within the range from 0.5 to < 8 Hz reached its maximum after that in the range from 7.5 to 12.5 Hz.

During the first 5 min in which the MOAA/S score was ≤ 1 (from about 5 to 10 min after the start of the infusion) the absolute power in the range of 0.5 to 3.5 Hz (Figure 15A) was not higher than about 200 µV², and that in the range of 3.5 to 7.5 Hz (Figure 16A) was not higher than about 25 µV². The absolute power in the range of 7.5 to 12.5 Hz (Figure 17A) was not lower than about 70 µV², and that in the range >12.5 Hz (Figure 18A) was not lower than about 80 µV². Thus, it was found that the absolute power in the range of 3.5 to 7.5 Hz was lower than that in the range of 7.5 to 12.5 Hz and lower than that in the range> 12.5 Hz during the first 5 min in which the MOAA/S score was ≤ 1, and it remained lower than those at any time at least during the first 35 min in which the MOAA/S score was ≤ 1.

When the MOAA/S score returned to a value of > 1 (about 45 min after the start of infusion), the absolute power in the range of 0.5 to 3.5 Hz (Figure 15A) was still considerably above 100 µV², whereas immediately before the point in time when a MOAA/S score of ≤ 1 was first reached (about 5 min after the start of infusion) it was lower than that.

During the first 5 min in which the MOAA/S score was ≤ 1 (from about 5 to 10 min after the start of the infusion), the absolute power in the range of > 12.5 Hz (Figure 18A) was not lower than about 100 µV², which was about the value immediately before the point in time when a MOAA/S score of ≤ 1 was first reached. This was the case at least during the first 10 min in which the MOAA/S score was ≤ 1.

The corresponding time courses of relative power are shown in Figures 15B, 16B, 17B and 18B. During the first 5 min in which the MOAA/S score was ≤ 1 (from about 5 to 10 min after the start of the infusion), the relative power in the range of 0.5 to 3.5 Hz (Figure 15B) was not higher than immediately before the point in time when a MOAA/S score of ≤ 1 was first reached (about 5 min after the start of infusion, at which point the relative power was about 40%). Only after 20 min after the start of the infusion (after about the first 15 min in which the MOAA/S score was ≤ 1) it reached values of about 50% or higher.

The relative power in the range of 3.5 to 7.5 Hz (Figure 16B) was not higher than about 7%, which was about the same relative power as immediately before the point in time when a MOAA/S score of ≤ 1 was first reached. It remained no higher than this value at any time when the MOAA/S score was ≤ 1.

## Claims

1. A benzodiazepine or pharmaceutically acceptable salt thereof for use in inducing and/or maintaining a MOAA/S score of ≤ 1 in a human patient selected from an infant, a child and a patient with a cognitive impairment that is at least mild cognitive impairment,
wherein the benzodiazepine or salt is defined by having the property of leading to one or more of the following characteristics of an electroencephalogram of a human reference subject in case said benzodiazepine or salt is administered to said reference subject to induce and/or maintain a MOAA/S score of ≤ 1:
(a) the absolute power in a frequency region selected within the range from 0.5 to < 8 Hz reaches its maximum after the first 5 min in which the MOAA/S score is ≤ 1,
(b) after the point in time when a MOAA/S score of ≤ 1 is first reached, the absolute power in a frequency region selected within the range from 0.5 to < 8 Hz reaches its maximum after that in the range from 8 to < 13 Hz, that in the range from 7.5 to 12.5 Hz or that in the range from 13 to < 30 Hz,
(c) the absolute power in a frequency region selected within the range from 0.5 to < 8 Hz is lower than that in the range from 7.5 to 12.5 Hz, that in the range from 8 to < 13 Hz, that in the range of > 12.5 Hz or that in the range from 13 to < 30 Hz during the first 5 min in which the MOAA/S score is ≤ 1,
(d) during the first 5 min in which the MOAA/S score is ≤ 1, the relative power in a frequency region selected within the range from 0.5 to < 8 Hz is not higher than 140% of that immediately before the point in time when a MOAA/S score of ≤ 1 is first reached, wherein the relative power in said frequency region indicates the absolute power in said frequency region in relation to the absolute power summed over the range from 0.5 to < 30 Hz,
(e) when the MOAA/S score returns to a value of > 1, the absolute power in a frequency region selected within the range from 0.5 to < 8 Hz has not yet returned to that immediately before the point in time when a MOAA/S score of ≤ 1 is first reached.

2. The benzodiazepine or salt for use according to claim 1, wherein the patient is further characterised as follows:
(i) the infant is up to 9 months of age,
(ii) the child is 1 to 9 years of age, and/or
(iii) the patient with the cognitive impairment is at least 65 years of age.

3. A benzodiazepine or pharmaceutically acceptable salt thereof for use in inducing and/or maintaining a MOAA/S score of ≤ 1 in a patient without causing brain damage,
wherein the benzodiazepine or salt is defined by having the property of leading to one or more of the following characteristics of an electroencephalogram of a human reference subject in case said benzodiazepine or salt is administered to said reference subject to induce and/or maintain a MOAA/S score of ≤ 1:
(a) the absolute power in a frequency region selected within the range from 0.5 to < 8 Hz reaches its maximum after the first 5 min in which the MOAA/S score is ≤ 1,
(b) after the point in time when a MOAA/S score of ≤ 1 is first reached, the absolute power in a frequency region selected within the range from 0.5 to < 8 Hz reaches its maximum after that in the range from 8 to < 13 Hz, that in the range from 7.5 to 12.5 Hz or that in the range from 13 to < 30 Hz,
(c) the absolute power in a frequency region selected within the range from 0.5 to < 8 Hz is lower than that in the range from 7.5 to 12.5 Hz, that in the range from 8 to < 13 Hz, that in the range of > 12.5 Hz or that in the range from 13 to < 30 Hz during the first 5 min in which the MOAA/S score is ≤ 1,
(d) during the first 5 min in which the MOAA/S score is ≤ 1, the relative power in a frequency region selected within the range from 0.5 to < 8 Hz is not higher than 140% of that immediately before the point in time when a MOAA/S score of ≤ 1 is first reached, wherein the relative power in said frequency region indicates the absolute power in said frequency region in relation to the absolute power summed over the range from 0.5 to < 30 Hz,
(e) when the MOAA/S score returns to a value of > 1, the absolute power in a frequency region selected within the range from 0.5 to < 8 Hz has not yet returned to that immediately before the point in time when a MOAA/S score of ≤ 1 is first reached.

4. The benzodiazepine or salt for use according to any of the preceding claims, which is defined by having said property in case said benzodiazepine or salt is administered to said reference subject under one or more of the following conditions:
(I) continuous intravenous administration of the benzodiazepine or salt,
(II) administration of the benzodiazepine or salt at a rate of 5 mg/min for 5 min, followed by a rate of 3 mg/min for 15 min and of 1 mg / min for another 15 min,
(III) electrode placement on the reference subject's scalp according to the international 10/20 system; frontal lead F3-Cz and Fp1 as ground,
(IV) recording the electroencephalogram with a Nicolet v32 amplifier and NeuroWorks 8 software,
(V) continuous recording of the electroencephalogram, digitisation at a sampling rate of 500 Hz with a gain of 0.1525902 µV/unit, first low-pass filtered via a finite impulse response FIR at 47 Hz, down sampled to 125 Hz, and subsequently high-pass filtered with a FIR filter at 0.5 Hz and segmented into EEG epochs of 8.192 s (2¹⁰ data points) length,
(VI) placement of three EEG electrodes on the reference subject's forehead with a distance of at least 8 cm between the two signal electrodes,
(VII) recording the electroencephalogram with a Narcotrend-Compact M monitor,
(VIII) reference subject's age of 20 to 38 years,
(IX) reference subject's body weight of 64 to 99 kg,
(X) reference subject's body mass index of 21 to 29 kg/m²,
(XI) reference subject's height of 169 to 197 cm,
(XII) reference subject is a male.

5. The benzodiazepine or salt for use according to any of the preceding claims, which is further defined by having the additional property of leading to the following characteristic of the electroencephalogram of said reference subject when administered as defined in claim 1: during the first 5 min in which the MOAA/S score is ≤ 1, the absolute power in the range from 13 to < 30 Hz, in the range of > 12.5 Hz, or in the range from 30 to 50 Hz is not lower than that immediately before the point in time when a MOAA/S score of ≤ 1 is first reached.

6. The benzodiazepine or salt for use according to any of the preceding claims, wherein said use involves
- administration to the patient by intravenous infusion or
- intrapulmonary and/or intranasal administration to the patient.

7. The benzodiazepine or salt for use according to any of the preceding claims,
wherein
- in characteristic (a), (b), (c), (d) and (f) the absolute power is a time-average over 10 s and in characteristic (e) the relative power is a time-average over 10 s , and/or
- the frequency region selected within the range from 0.5 to < 8 Hz is 0.5 to 3.5 Hz, 3.5 to 7.5 Hz, 0.5 to < 2 Hz, 0.5 to 2 Hz, 2 to < 5 Hz, 2 to 5 Hz, 5 to < 8 Hz, 2 to < 8 Hz, 0.5 to < 3 Hz or 0.5 to 5 Hz.

8. The benzodiazepine or salt for use according to any of the preceding claims, wherein the benzodiazepine is according to formula (I) 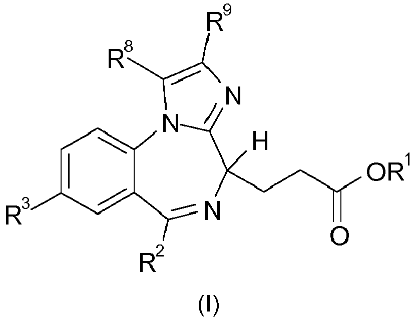 wherein
R¹ is CH₃, CH₂CH₃, CH₂CH₂CH₃, CH(CH₃)₂ or CH₂CH(CH₃)₂;
R² is 2-fluorophenyl, 2-chlorophenyl or 2-pyridyl;
R³ is Cl or Br;
R⁸ is hydrogen, C₁₋₄ alkyl or C₁₋₃ hydroxyalkyl; and
R⁹ is H, C₁₋₄ alkyl, C₁₋₃ hydroxyalkyl or C₁₋₄ alkoxy.

9. The benzodiazepine or salt for use according to claim 8, which is methyl 3-[(4S)-8-bromo-1-methyl-6-(pyridin-2-yl)-4H-imidazo[1,2-a][1,4]benzodiazepin-4-yl]propanoate.

10. The benzodiazepine or salt for use according to claim 9, wherein the use involves administration
- at a dose of 0.3 to 40 mg/h/kg or
- at a fixed dose irrespective of the patient's body weight.

11. The benzodiazepine or salt for use according to any of the preceding claims, wherein the salt is selected from the group consisting of halogenides, sulfate, organic sulfates, sulfonate, organic sulfonates, nitrate, phosphate, salicylate, tartrate, citrate, maleate, formiate, malonate, succinate, isethionate, lactobionate and sulfamate.

12. The benzodiazepine or salt for use according to any of the preceding claims, which is used together with a further active ingredient or an excipient.

13. The benzodiazepine or salt for use according to claim 12, wherein
- the further active ingredient is an opioid or
- the excipient is a disaccharide and/or a dextran.
